# EUROPEAN PATENT SPECIFICATION

(11) **EP 3 124 620 B1**
(45) Date of publication and mention of the grant of the patent: **08.08.2018**
(21) Application number: 15306219.5
(22) Date of filing: 27.07.2015
(51) Int. Cl.: C12Q 1/6876

(54) **ACTIVATORS OF UC.291 FOR USE FOR IMPROVING SKIN BARRIER FUNCTION AND/OR FOR PREVENTING AND/OR ATTENUATING SKIN AGEING AND/OR FOR HYDRATING SKIN**
AKTIVATOREN VON UC.291 ZUR VERWENDUNG ZUR VERBESSERUNG DER HAUTBARRIEREFUNKTION UND/ODER ZUR PRÄVENTION UND/ODER ABSCHWÄCHUNG VON HAUTALTERUNG UND/ODER ZUR HYDRATISIERUNG DER HAUT
ACTIVATEURS DE UC. 291 DESTINÉ À ÊTRE UTILISÉ POUR AMÉLIORER LA FONCTION BARRIÈRE DE LA PEAU ET/OU POUR PRÉVENIR ET/OU ATTÉNUER LE VIEILLISSEMENT DE LA PEAU ET/OU POUR HYDRATER LA PEAU

(43) Date of publication of application: 01.02.2017
(73) Proprietor: Chanel Parfums Beauté, 92200 Neuilly-sur-Seine (FR)
(72) Inventor: CANDI, Eleonora, 00118 Rome (IT); MELINO, Gerry, 00141 Rome (IT); SAINTIGNY, Gaëlle, 75019 PARIS (FR); MAHE, Christian, 92200 Neuilly sur Seine (FR)
(74) Representative: Cabinet Plasseraud

(56) References cited:
- WO-A1-2012/156420
- FR-A1- 2 987 371
- Anonymous: "Anno accademico 2012/2013. Dottorato in Biochimica e Biologia Molecolare.", , February 2014 (2014-02), XP055237865, Retrieved from the Internet: URL:http://dottoratobiochimica.uniroma2.it /files/2014/02/annual-progress-report-2013 .pdf [retrieved on 2015-12-21]
- TAEKO MIZUTANI ET AL: "Anti-photoaging capability of antioxidant extract from Camellia japonica leaf", EXPERIMENTAL DERMATOLOGY, vol. 23, 1 October 2014 (2014-10-01), pages 23-26, XP055237882, COPENHAGEN; DK ISSN: 0906-6705, DOI: 10.1111/exd.12395
- MARTYNA MORUS ET AL: "Plant stem cells as innovation in cosmetics", ACTA POLONIAE PHARMACEUTICA - DRUG RESEARCH, vol. 71, no. 5, 1 October 2014 (2014-10-01), pages 701-707, XP055237799, PL ISSN: 0001-6837
- BEJERANO GILL ET AL: "Ultraconserved elements in the human genome", SCIENCE, AMERICAN ASSOCIATION FOR THE ADVANCEMENT OF SCIENCE, US, vol. 304, no. 5675, 28 May 2004 (2004-05-28), pages 1321-1325, XP002601779, ISSN: 0036-8075, DOI: 10.1126/SCIENCE.1098119 & Gill Bejerano ET AL: "Ultra-conserved elements in the human genome Authors and affiliations. Supporting online material", SCIENCE Express, 6 May 2004 (2004-05-06), XP055238215, Retrieved from the Internet: URL:http://www.sciencemag.org/content/supp l/2004/05/27/1098119.DC1/Bejerano.SOM.pdf [retrieved on 2015-12-23]

## Description

The invention relates to the identification and the use of compounds which activate the expression or activity of uc.291 for improving skin barrier function, and/or for preventing and/or attenuating ageing, and/or for hydrating skin.

Non-coding RNAs (ncRNA, transcript >200 nucleotides) play important biological functions, they are expressed in a tissue specific manner and are involved in physiological and pathological conditions in many organs including skin. ncRNAs are a broad class of transcripts consisting of structural (such as ribosomal RNAs (rRNAs), transfer RNAs (tRNAs), small nuclear RNAs (snRNAs), or small nucleolar RNAs (snoRNAs)), regulatory (such as miRNAs, or piwi-interacting RNAs (piRNAs)), and of sense/antisense transcripts, whose functions remain mostly uncharacterized. While a number of transcripts falling in that category have been studied for some time (e.g. Xist), the realization that long non-coding RNAs (lncRNAs) represent a very abundant RNA subclass is relatively recent and originates from genome-wide transcriptome studies (Wang et al, 2011).

lncRNAs are estimated to be at least as abundant as mRNAs, and like mRNAs, their expression levels and patterns are often tissue-specific. The heterogeneity in sequence, structure and size, along with the widely different subcellular localizations of lncRNAs points to their involvement in numerous cellular processes (Clark et al, 2011). In the nucleus, a few lncRNAs were shown to inhibit or activate transcription, which is in contrast to other ncRNAs thought to represent basic features of transcription. Conversely, chromatin conformation is thought to play a role in coordinating gene expression with lncRNAs (Wang et al, 2011). There are several evidences supporting the fact also in epidermis development and keratinocyte differentiation, wherein chromatin-remodelling and transcription machinery are expressed in highly organized manner to establish tissue-specific patterns of gene activation and silencing (Fessing et al, 2011).

Considering the skin tissue, which constitutes a barrier against external attack, such as UV rays, there always remains the need to propose active agents for maintaining this barrier effective, and thus for maintaining the tissue-specific pattern of gene activation and silencing. The skin consists mainly of three layers, namely, starting from the uppermost layer, the epidermis, the dermis and the hypodermis. The epidermis in particular consists of keratinocytes (predominantly), melanocytes (involved in pigmenting the skin) and Langerhans cells. Its function is to protect the body from the external environment and to ensure its integrity, and especially to prevent the penetration of microorganisms or chemical substances, and the evaporation of the water contained in the skin.

To do this, keratinocytes undergo a process of proliferation and then of continuous directed maturation during which the keratinocytes located in the basal layer of the epidermis form, at the final stage of their differentiation, corneocytes, which are totally keratinized dead cells in the form of horny sheaths consisting of proteins and lipids such as epidermal ceramides. During this differentiation process, intercorneocytic epidermal lipids are also formed and then organized in the form of bilayers (lamellae) in the *stratum corneum,* and they participate, with the abovementioned horny sheaths, in the barrier function of the epidermis.

The barrier function of the epidermis may, however, be perturbed under certain climatic conditions (for example under the effect of cold and/or the wind) or under the effect of stress or fatigue, especially, thus promoting the penetration of allergens, irritants or microorganisms. These external factors may lead to drying of the skin (the skin loses its permeability, becomes dehydrated and its transepidermal water loss increases), and sensations of heating or redness, and also to impair the radiance of the complexion and the suppleness of the skin. Impairment of the skin barrier may also promote the appearance of microchapping or microcracks. Furthermore, a badly formed barrier, resulting from impaired proliferation and differentiation processes, no longer protects the skin against UV radiation or any other type of external attack. The UV rays penetrating the skin may then produce free radicals which may have a detrimental effect on various targets, such as activate collagenases and elastases which are responsible for the degradation of collagen and elastin, respectively, and thus for a decrease in skin elasticity and firmness and the formation of wrinkles.

To prevent or correct this phenomenon, it is known practice to apply to the skin cosmetic compositions containing hygroscopic agents, such as sugars or polyols, which are intended to take up the water present in the skin and thus to impede its evaporation. Use has also conventionally been made of fatty substances that allow an occlusive film to be formed on the skin, which contributes towards impeding the evaporation of water. Moreover, these compositions frequently incorporate active agents that act on one or more of the various biological targets involved either in skin turnover processes, in particular in keratinocyte differentiation, epidermal lipid synthesis and corneocyte cohesion, or in the endogenous synthesis of natural moisturizing factor (NMF) constituents of the skin, in particular in the synthesis of proteoglycans.

However, there always remains the need to propose novel cosmetic active agents for reinforcing the skin's barrier function to prevent and/or reduce the sensations of cutaneous discomfort, stinging, tautness, itching, sensations of heating or redness and/or the appearance of microchapping or microcracking and/or the loss of radiance of the complexion or dull complexion and/or the loss of suppleness of the skin and/or to improve the protection of the epidermis against UV. WO 2012/156420 discloses an in vitro method for screening for candidate compounds for preventing and/or attenuating ageing of the skin, and/or for hydrating the skin, comprising measuring the expression of at least one gene selected from LARGE, HS6ST2 and ST8SIA1 in keratinocytes exposed to the compound. The inventors have surprisingly identified a lncRNA specifically upregulated during keratinocyte differentiation. This lncRNA belongs to a family which is transcribed from regions that exhibit extremely high conservation between the orthologous regions of human, rat and mouse genomes (Bejerano et al 2004a; Bejerano et al, 2004b), and called 'transcribed-ultraconserved' regions (T-uc) or ultraconserved genes, and these transcripts range from 100-200 nucleotides (Calin et al, 2007). Their striking evolutionary retention suggests profound biological roles in a wide variety of physiologic responses.

This lncRNA is uc.291, which is a pro-differentiation lncRNA. Keratinocytes lacking uc.291 show a delay in keratinocyte differentiation and an increase in proliferation. As shown in the Examples, uc.291 is highly present in the nucleus of the keratinocytes and works as transcriptional enhancer of surfactant protein D (SFTPD), Zinc-finger MIZ type (ZMIZ1) and zinc finger SWIM type containing 8 (ZSWIM8).

This lncRNA uc.291 may be used as a marker of the state of skin barrier function.

Thus, the present invention provides a method for identifying useful agents for improving skin barrier function, and/or for preventing and/or attenuating ageing of the skin, and/or for hydrating the skin, by using said uc.291.

The present invention thus relates to an *in vitro* method for screening for candidate compounds for improving skin barrier function, and/or for preventing and/or attenuating ageing of the skin, and/or for hydrating the skin, comprising the following steps:
a. bringing at least one test compound into contact with a sample of keratinocytes ;
b. measuring the expression or the activity of uc.291 in said keratinocytes;
c. selecting the compounds for which an activation of at least 20%, preferably at least 30%, preferably at least 40% of the expression or an activation of at least 20%, preferably at least 30%, preferably at least 40% of the activity of uc.291 is measured in the keratinocytes treated in a. compared with the untreated keratinocytes.

According to a first embodiment, step b. is performed before and after step a. In this case, the expression or activity of uc.291 measured in the keratinocytes before step a. corresponds to the control value (i.e. untreated keratinocytes). Thus, step c. comprises the selection of the compounds for which an activation of at least 20%, preferably at least 30%, preferably at least 40% of the expression or an activation of at least 20%, preferably at least 30%, preferably at least 40% of the activity of uc.291 is measured in the keratinocytes treated in a. compared with the same keratinocytes before step a.

According to another embodiment, the method comprises a first step a'. of preparing samples of keratinocytes. Thus, preferably, the present invention relates to an *in vitro* method for screening for candidate compounds for improving skin barrier function, and/or for preventing and/or attenuating ageing of the skin, and/or for hydrating the skin, comprising the following steps:
a'. preparing at least two samples of keratinocytes, preferably pre-senescent;
a. bringing one of the samples into contact with at least one test compound ; then
b. measuring the expression or the activity of uc.291 in said samples ; and
c. selecting the compounds for which an activation of at least 20%, preferably at least 30%, preferably at least 40% of the expression or an activation of at least 20%, preferably at least 30%, preferably at least 40% of the activity of uc.291 is measured in the keratinocytes treated in a. compared with the sample of untreated keratinocytes.

In this second embodiment, the expression or activity of uc.291 measured in the sample of keratinocytes not submitted to step a. corresponds to the control value (i.e. untreated keratinocytes).

By the expression "ageing of the skin" is intended any change in the external appearance of the skin due to ageing, whether this is chronobiological and/or photo-induced, such as, for example, wrinkles and fine lines, withered skin, flaccid skin, thinned skin, and skin lacking elasticity and/or tonus, and also any internal change in the skin which is not systematically reflected by a changed external appearance, such as, for example, any internal degradation of the skin, particularly of collagen, following exposure to ultraviolet radiation.

By "hydrating the skin", it is meant maintaining the natural humidity of the skin and preventing its drying.

"uc.291" or "lncRNA uc.291" is the ultraconserved non-coding elements (database UCNE: http://ccg.vital-it.ch/UCNEbase/) with code name ZNF503_Siddhartha (http://ccg.vital-it.ch/UCNEbase/list.php?data=ucne&org=hg19&view=uceID&value=uc.291). The sequence of uc.291 is SEQ ID NO:1 below:

The test compounds tested may be of any type. They may be of natural origin or may have been produced by chemical synthesis. This may involve a library of structurally defined chemical compounds, uncharacterized compounds or substances, or a mixture of compounds.

Natural compounds include compounds from vegetal origin, like plants. Preferably, the test compounds are vegetal, preferably chosen from botanical extracts.

According to step a., the test compound is put into contact with a sample of keratinocytes.

According to step b., the expression and/or the activity of uc.291 is measured in said keratinocytes.

The term "expression of uc.291" is intended to mean the amount of produced uc.291.

The term "activity of uc.291" is intended to mean the ability of uc.291 to enhance the level of transcription of the portion of sequence to which it hybridizes.

Those skilled in the art are familiar with the techniques for quantitatively or semi-quantitatively detecting the sequence to which uc.291 hybridizes, and thus, determining said uc.291 activity. Techniques based on hybridization of the sequence with specific nucleotide probes are the most common, like Northern blotting, RT-PCR (reverse transcriptase polymerase chain reaction), quantitative RT-PCR (qRT-PCR).

Those skilled in the art are also familiar with the techniques for quantitatively or semi-quantitatively detecting uc.291, or the sequence to which uc.291 hybridizes. In particular, the expression of uc.291 can be measured by real-time PCR. The activity of uc.291 can be measured by real-time PCR on the sequence targets, or by evaluating the protein level of the target by Western blot.

Preferably, the expression of uc.291 is measured by real-time PCR.

The expression or activity of uc.291 after treatment with the test compound is then compared to a control value, i.e. a value obtained in the same keratinocytes before treatment, or a value obtained in another sample of keratinocytes which are untreated.

According to step c., the useful compounds are those for which an activation of at least 20%, preferably at least 30%, preferably at least 40% of the expression or an activation of at least 20%, preferably at least 30%, preferably at least 40% of the activity of uc.291 is measured in the treated keratinocytes, compared with untreated keratinocytes. Preferably, the activation of the expression or of the activity of uc.291 is of at least 50%, preferably of at least 60%.

The compounds selected by means of the screening methods defined herein can subsequently be tested on other *in vitro* models and/or *in vivo* models (in animals or humans) for their effects on skin barrier function and/or skin ageing and/or skin hydration. The useful compounds according to the disclosure are activators of the targeted uc.291.

A subject of the disclosure is also the cosmetic use of an activator of uc.291, said activator being identified according to the above described method, for improving skin barrier function and/or for preventing and/or attenuating ageing of the skin and/or for hydrating the skin.

According to another aspect, an object of the present disclosure is the use of at least one uc.291 activator, said activator being identified according to the above described method, to make a therapeutic composition for improving skin barrier function and/or for preventing and/or attenuating ageing of the skin and/or for hydrating the skin. The present disclosure thus also relates to the use of a uc.291 activator, said activator being identified according to the above described method, for improving skin barrier function and/or for preventing and/or attenuating ageing of the skin and/or for hydrating the skin.

The activator refers to a compound which substantially increases the expression or activity of uc.291. The term "substantially" signifies an increase of at least 20%, preferably at least 30%, preferably at least 40%, preferably of at least 50%, and more preferably of at least 60%.

The uc.291 activator can be used in a proportion of from 0.001 to 10% by weight, preferably in a proportion of from 0.01 to 5% by weight of the composition.

The uc.291 activators identified thanks to the screening method described above can be formulated within a composition, in combination with a physiologically acceptable carrier, preferably a cosmetically acceptable medium, i.e. a medium that is suitable for use in contact with human skin without any risk of toxicity, incompatibility, instability or allergic response and especially that does not cause any sensations of discomfort (redness, tautness, stinging, etc.) that are unacceptable to the user. These compositions may be administered, for example, orally, or topically. Preferably, the composition is applied topically. By oral administration, the composition may be in the form of tablets, gel capsules, sugar-coated tablets, syrups, suspensions, solutions, powders, granules, emulsions, suspensions of microspheres or nanospheres or lipid or polymeric vesicles for controlled release. By topical administration, the composition is more particularly for use in treating the skin and the mucous membranes and may be in the form of salves, creams, milks, ointments, powders, impregnated pads, solutions, gels, sprays, lotions or suspensions. It may also be in the form of suspensions of microspheres or nanospheres or lipid or polymeric vesicles or polymeric patches or hydrogels for controlled release. This composition for topical application may be in anhydrous form, in aqueous form or in the form of an emulsion. The composition for topical application may be in the form of an oil-in-water, water-in-oil or multiple emulsion (W/O/W or O/W/O), which may optionally be microemulsions or nanoemulsions, or in the form of an aqueous dispersion, a solution, an aqueous gel or a powder. In a preferred variant, the composition is in the form of a gel, a cream or a lotion.

The physiologically acceptable carrier of the composition generally comprises water and optionally other solvents such as ethanol.

This composition is preferably used as a care and/or cleansing product for facial and/or bodily skin and it may especially be in the form of a fluid, a gel or a mousse, conditioned, for example, in a pump-dispenser bottle, an aerosol or a tube, or in the form of cream conditioned, for example, in a jar. As a variant, it may be in the form of a makeup product and in particular a foundation or a loose or compact powder.

It may comprise various adjuvants, such as at least one compound chosen from:
- oils, which may be chosen especially from: linear or cyclic, volatile or nonvolatile silicone oils, such as polydimethylsiloxanes (dimethicones), polyalkylcyclosiloxanes (cyclomethicones) and polyalkylphenylsiloxanes (phenyl dimethicones); synthetic oils such as fluoro oils, alkylbenzoates and branched hydrocarbons such as polyisobutylene; plant oils and especially soybean oil or jojoba oil; and mineral oils such as liquid petroleum jelly;
- waxes such as ozokerite, polyethylene wax, beeswax or carnauba wax;
- silicone elastomers obtained especially by reaction, in the presence of a catalyst, of a polysiloxane containing at least one reactive group (especially hydrogen or vinyl) and bearing at least one alkyl group (especially methyl) or phenyl, in a terminal and/or side position, with an organosilicone such as an organohydrogenopolysiloxane;
- surfactants, preferably emulsifying surfactants, whether they are nonionic, anionic, cationic or amphoteric, and in particular fatty acid esters of polyols such as fatty acid esters of glycerol, fatty acid esters of sorbitan, fatty acid esters of polyethylene glycol and fatty acid esters of sucrose; fatty alkyl ethers of polyethylene glycol; alkylpolyglucosides; polysiloxane-modified polyethers; betaine and derivatives thereof; polyquaterniums; ethoxylated fatty alkyl sulfate salts; sulfosuccinates; sarcosinates; alkyl and dialkyl phosphates, and salts thereof; and fatty acid soaps;
- co-surfactants such as linear fatty alcohols and in particular cetyl alcohol and stearyl alcohol;
- thickeners and/or gelling agents, and in particular crosslinked or non-crosslinked, hydrophilic or amphiphilic homopolymers and copolymers, of acryloylmethylpropanesulfonic acid (AMPS) and/or of acrylamide and/or of acrylic acid and/or of acrylic acid salts or esters; xanthan gum or guar gum; cellulose derivatives; and silicone gums (dimethiconol);
- organic screening agents, such as dibenzoylmethane derivatives (including butyl-methoxydibenzoylmethane), cinnamic acid derivatives (including ethylhexyl methoxycinnamate), salicylates, para-aminobenzoic acids, β,β'-diphenyl acrylates, benzophenones, benzylidenecamphor derivatives, phenylbenzimidazoles, triazines, phenylbenzotriazoles and anthranilic derivatives;
- inorganic screening agents, based on mineral oxides in the form of coated or uncoated pigments or nanopigments, and in particular based on titanium dioxide or zinc oxide;
- dyes;
- preserving agents;
- sequestrants such as EDTA salts;
- fragrances;
- and mixtures thereof, without this list being limiting.

Examples of such adjuvants are especially mentioned in the CTFA dictionary (International Cosmetic Ingredient Dictionary and Handbook published by The Cosmetic, Toiletry and Fragrance Association, 11th edition, 2006), which describes a wide variety, without limitation, of cosmetic and pharmaceutical ingredients usually used in the skincare industry, that are suitable for use as additional ingredients in the compositions according to the present disclosure. The composition may also comprise at least one compound with an optical effect such as fillers, pigments, nacres, tensioning agents and matting polymers, and mixtures thereof.

The term "fillers" should be understood as meaning colorless or white, mineral or synthetic, lamellar or non-lamellar particles suitable for giving the composition body or rigidity and/or softness, a matt effect and uniformity immediately on application. Fillers that may especially be mentioned include talc, mica, silica, kaolin, Nylon® powders such as Nylon-12 (Orgasol® sold by the company Atochem), polyethylene powders, polyurethane powders, polystyrene powders, polyester powders, optionally modified starch, silicone resin microbeads such as those sold by the company Toshiba under the name Tospearl®, hydroxyapatite, and hollow silica microspheres (Silica Beads® from the company Maprecos).

The term "pigments" should be understood as meaning white or colored, mineral or organic particles that are insoluble in the medium, which are intended to color and/or opacify the composition. They may be of standard or nanometric size. Among the mineral pigments that may be mentioned are titanium dioxide, zirconium dioxide and cerium dioxide, and also zinc oxide, iron oxide and chromium oxide.

The term "nacres" should be understood as meaning iridescent particles that reflect light. Among the nacres that may be envisaged, mention may be made of natural mother-of-pearl, mica coated with titanium oxide, with iron oxide, with natural pigment or with bismuth oxychoride, and also colored titanium mica.

The mass concentration in the aqueous phase of these fillers and/or pigments and/or nacres is generally from 0.1% to 20% and preferably from 0.2% to 7% by weight relative to the total weight of the composition.

The term "tensioning agent" should be understood as meaning a compound suitable for making the skin taut and, by means of this tension effect, making the skin smooth and reducing or even immediately eliminating wrinkles and fine lines therefrom. Tensioning agents that may be mentioned include polymers of natural origin. The term "polymer of natural origin" means polymers of plant origin, polymers derived from integuments, egg proteins and latices of natural origin. These polymers are preferably hydrophilic. Polymers of plant origin that may especially be mentioned include proteins and protein hydrolyzates, and more particularly extracts of cereals, of legumes and of oil-yielding plants, such as extracts of corn, of rye, of wheat, of buckwheat, of sesame, of spelt, of pea, of bean, of lentil, of soybean and of lupin. The synthetic polymers are generally in the form of a latex or a pseudolatex and may be of polycondensate type or obtained by free-radical polymerization. Mention may be made especially of polyester/polyurethane and polyether/polyurethane dispersions. Preferably, the tensioning agent is a copolymer of PVP/dimethiconyl acrylate and of hydrophilic polyurethane (Aquamere S-2001® from the company Hydromer).

The term "matting polymers" means herein any polymer in solution, in dispersion or in the form of particles, which reduces the sheen of the skin and which unifies the complexion. Examples that may be mentioned include silicone elastomers; resin particles; and mixtures thereof. Examples of silicone elastomers that may be mentioned include the products sold under the name KSG® by the company Shin-Etsu, under the name Trefil®, BY29® or EPSX® by the company Dow Corning or under the name Gransil® by the company Grant Industries.

The composition used according to the disclosure may also comprise active agents other than the uc.291 activator, and in particular at least one active agent chosen from: agents that stimulate the production of growth factors; anti-glycation or deglycating agents; agents that increase collagen synthesis or that prevent its degradation (anti-collagenase agents and especially matrix metalloprotease inhibitors); agents that increase elastin synthesis or prevent its degradation (anti-elastase agents); agents that stimulate the synthesis of integrin or of focal adhesion constituents such as tensin; agents that increase the synthesis of glycosaminoglycans or proteoglycans or that prevent their degradation (anti-proteoglycanase agents); agents that increase fibroblast proliferation; depigmenting or anti-pigmenting agents; antioxidants or free-radical scavengers or anti-pollution agents; and mixtures thereof, without this list being limiting.

Examples of such agents are especially: plant extracts and in particular extracts of *Chondrus crispus,* of *Thermus thermophilus,* of *Pisum sativum* (Proteasyl® TP LS), of *Centella asiatica,* of Scenedesmus, of *Moringa pterygosperma,* of witch hazel, of *Castanea sativa,* of *Hibiscus sabdriffa,* of *Polianthes tuberosa,* of *Argania spinosa,* of *Aloe vera,* of *Narcissus tarzetta,* or of liquorice; an essential oil of *Citrus aurantium* (Neroli); a-hydroxy acids such as glycolic acid, lactic acid and citric acid, and esters thereof; β-hydroxy acids such as salicylic acid and derivatives thereof; plant protein hydrolyzates (especially of soybean or of hazelnut); acylated oligopeptides (sold especially by the company Sederma under the trade names Maxilip®, Matrixyl® 3000, Biopeptide® CL or Biopeptide® EL); yeast extracts and in particular of *Saccharomyces cerevisiae;* algal extracts and in particular of laminairia; vitamins and derivatives thereof such as retinyl palmitate, ascorbic acid, ascorbyl glucoside, magnesium or sodium ascorbyl phosphate, ascorbyl palmitate, ascorbyl tetraisopalmitate, ascorbyl sorbate, tocopherol, tocopheryl acetate and tocopheryl sorbate; arbutin; kojic acid; ellagic acid; and mixtures thereof.

As a variant or in addition, the composition used according to the disclosure may comprise at least one elastase inhibitor (anti-elastase), such as an extract of *Pisum sativum* seeds that is sold especially by the company Laboratoires Sérobiologiques/Cognis France under the trade name Proteasyl TP LS®.

The composition may also contain inert additives or combinations of these additives, such as wetting agents, stabilizers, moisture regulators, pH regulators, osmotic pressure modifiers, or UV-A and UV-B screens.

The following examples illustrate the invention without limiting the scope thereof. These examples are based on the figures listed below:
**Figure 1****: uc.291 is expressed in keratinocytes during calcium-induced differentiation**
   Primary human keratinocytes were induced to differentiate by calcium addition (1.2 mM), for 3, 6 or 9 days.
   A) Evaluation of uc.291 during differentiation by RT-PCR, as positive controls (B) are used involucrin and K10 (differentiation markers).
**Figure 2****: Depletion of uc.291 delays differention**
   Depletion of uc.291 and evaluation by RT-PCR of the expression of differentiation markers K10 and involucrin mRNAs. Both markers are strongly reduced in uc.291 depleted cells in comparison to control.
**Figure 3****: Depletion of uc.291 increases proliferation**
   (A) Evaluation by RT-PCR of the silencing at 3 days post-transfection.
   (B) Cell cycle analysis of scramble transfected (Ctrl) and si-uc.291 transfected cells at 3 days post-transfection.
   (C) Evaluation by Western blot of proliferation marker (p63) and differentiation marker (K10) during calcium induced differentiation (1, 2, 3 days) upon uc.291 depletion.

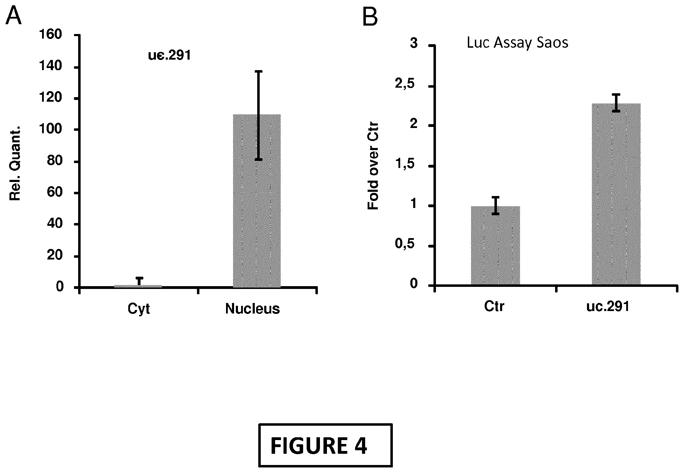

**Figure 4****: uc.291 is expressed in the nucleus and functions as enhancer**
   A) Biochemical fractionation of nucleus and cytosol of keratinocytes. Uc.291 is mainly detected in the nucleus.
   B) The conserved sequence of uc.291 was cloned in a vector for luciferase-assay, upstream of the promoter, and the inventors transfected this vector (uc.291) and the control vector (Ctr) in Saos-2 cells. Luciferase assay suggests that uc.291 acts as enhancer, since the luciferase activity increases two fold over control.

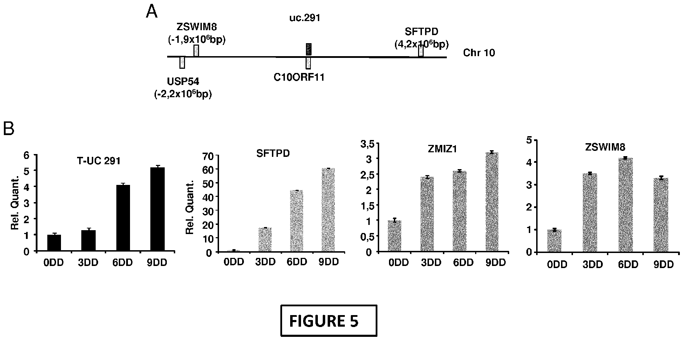

**Figure 5****: SFTP, ZMIZ1 and ZSWIM8 genes are localized in proximity of uc.291 and their expression is induced during differentiation**
   (A) Scheme of Chromosome 10 where uc.291 is located and the genes located within 4 millions of bases.
   (B) RT-PCRs showing that SFTPD, ZMIZ1 and ZSWIM8 genes expression parallel with uc.291 expression during differentiation induced by calcium.

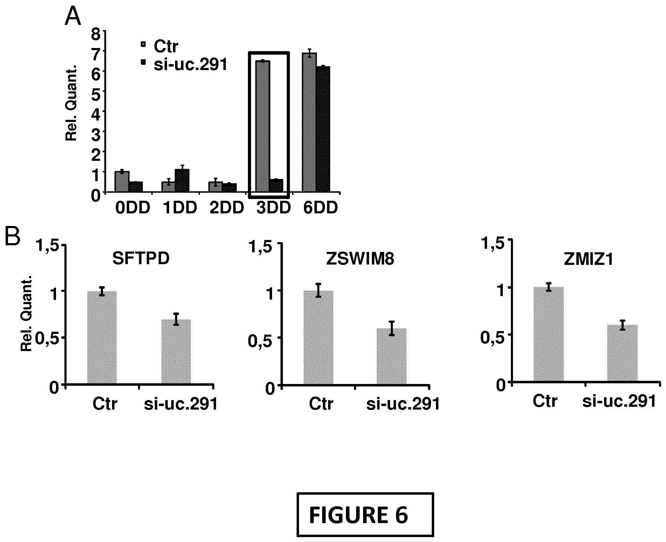

**Figure 6****: uc.291 expression enhances SFTPD expression. Silencing of SFTPD recapitulate si-uc.291 effect**
   (A) Keratinocytes were transfected with siRNA specific for uc.291 (si-uc.291) and treated with 1.2 mM calcium. Cells were analyzed at the indicated time points.
   (B) At 3 days the inventors evaluated the expression level of SFTPD, ZSWIM8 and ZMIZ1 by RT-PCR. The inventors found a 40% reduction in SFTPD, ZSWIM8 and ZMIZ1 mRNAs in absence of uc.291.

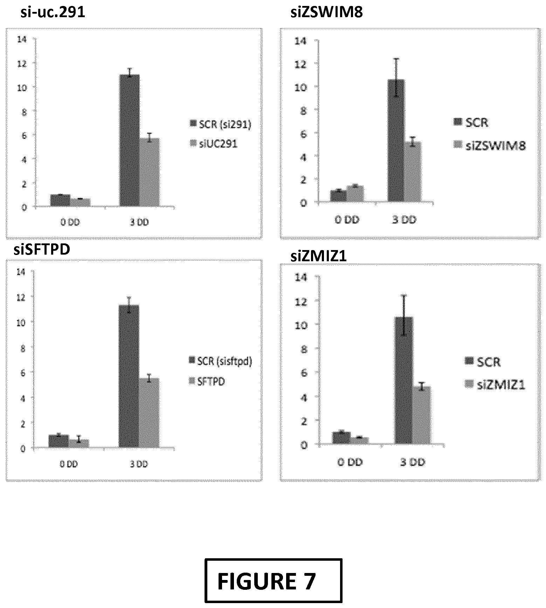

**Figure 7****: Silencing of SFTPD, ZMIZ1 and ZSWIM8 recapitulate si-uc.291 effect**
   Keratinocytes were transfected with siRNA specific for uc.291 (si-uc.291) and treated with 1.2 mM calcium for 3 days. The inventors evaluated the expression level of the differentiation marker involucrin. The inventors found a 40% reduction in involucrin expression mRNA upon si-SFTPD, si-ZSWIM8 and si-ZMIZ1, similarly to si-uc.291.

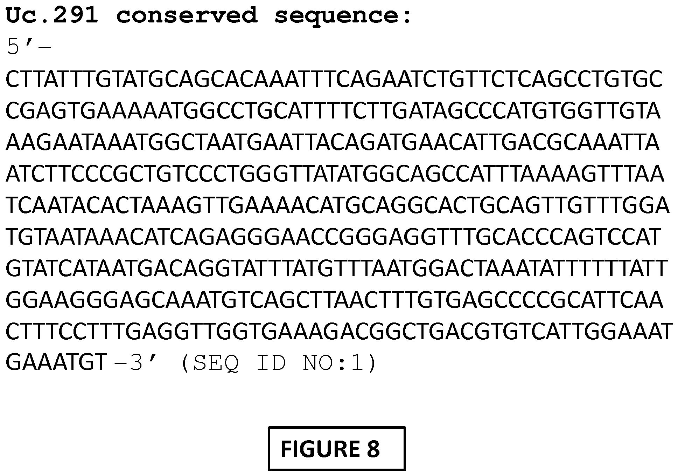

**Figure 8****: uc.291 sequence**
   Human uc.291 sequence 5' to 3'direction (database UCNE: http://ccg.vital-it.ch/UCNEbase/) with code name ZNF503_Siddhartha.

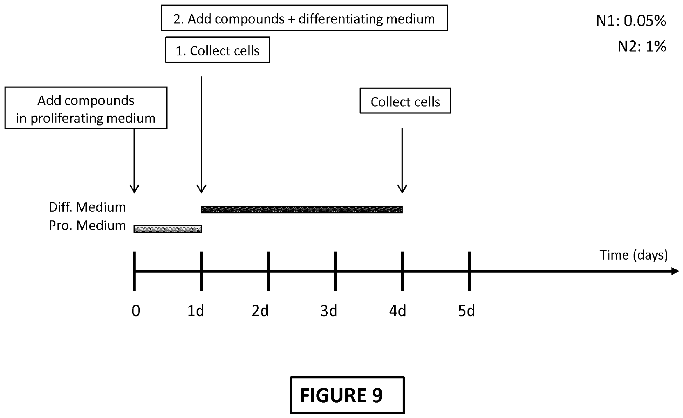

**Figure 9****: Experimental set-up for compounds**

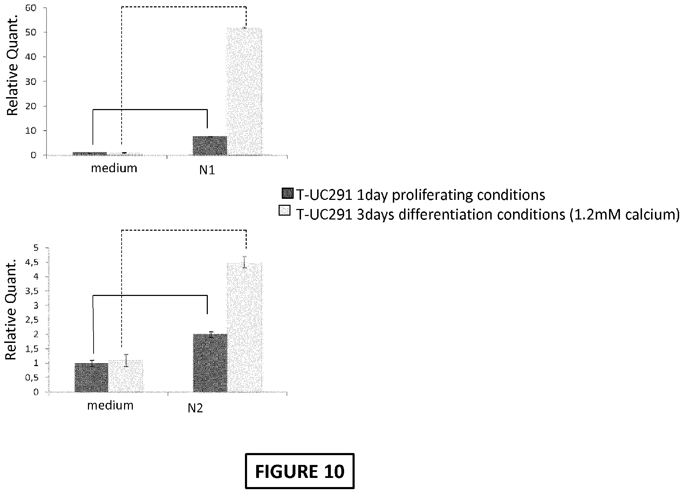

**Figure 10****: Different compound can modulate uc.291 expression**
   uc.291 expression was evaluated by real-time PCR upon treatments with compounds. The inventors can see that all the compounds have synergic effects with calcium in enhancing uc.291 expression.

### Example 1:

### Material and methods

### Cell culture and transfection

Human epidermal keratinocytes, neonatal (HEKn) (Cascade, Invitrogen) were grown in Epilife medium with HKGS growth supplements (Invitrogen) and cultured at 37°C in a humidified chamber with 5% CO₂. Cells were induced to differentiate *in vitro* by adding 1.2 mM CaCl₂ to the culture medium, then cells were grown in full confluence for up to 9 days. For uc.291 silencing, HEKn were transfected with si-291-1 HP Custom siRNA (Qiagen) and the transfection was performed using Lipofectamine RNAimax transfection reagent (Invitrogen) according to manufacturer's protocols. 48 hours after transfection, the medium was removed and calcium-induced differentiation was started by adding 1.2 mM CaCl₂ to the culture medium. Saos-2 cells were cultured in D-MEM F12 with 10% FBS, 100 penicillin, 100 µg/mL streptomycin (GIBCO, Invitrogen) and transfected with Lipofectamine 2000 according to manufacturer's protocols (Invitrogen).

### Cell culture and compounds

We have used the following compounds:
N1 : Camellia leaf extract: final concentration 0.05%
N2 : Stem cells of Camellia: final concentration 1%

Camellia leaf extract is prepared as follows : the extract of Camellia leaf is obtained by extraction of fresh and ground leaves of *Camellia japonica* with ethanol (or any alcoholic solvent), discoloration with activated charcoal, filtration and dilution with dipropylene glycol (or other appropriate cosmetic solvent) so as to obtain a final extract on a liquid form.

Stem cells of Camellia are stem cells obtained from meristems of *Camellia japonica alba plena* with the technology as disclosed in WO2003/077881.

The compounds were tested in proliferating and differentiation conditions. Cells (HEKn, 300.000/60mm dish) were plated in proliferating conditions medium and treated for 24 hours with the compounds at the indicated concentrations. Cells were collected after 1 day and analyzed for uc.291 expression by real-time PCR, as indicated below. Alternatively, differentiating medium (containing 1,2 mM calcium) was added to cells including the indicated compounds, and collected after 3 days to evaluate uc.291 expression level.

### RNA extraction and Real-Time PCR analysis

Total RNA was isolated from differentiated (days 3, 6 and 9) and proliferating Human Keratinocytes using the mirVana miRNA Isolation Kit (Ambion) following the manufacturer's protocol. One microgram of total RNA was reverse transcribed with GoScript™ Reverse Transcription System (Promega) according to manufacturer's protocols. Real Time PCR was then performed by using the Platinum SYBR Green qPCR Master Mix (Promega). The expression of each gene and uc.291 was defined from the threshold cycle (Ct) and relative expression levels were calculated by using the 2^{-ΔΔCt} method after normalization with reference to expression of housekeeping genes. Uc.291 conserved sequence is shown in Figure 8.

### Cell proliferation and cell cycle analysis

Incorporation of bromodeoxyuridine (BrdU) during DNA synthesis was evaluated with the Click-iT™ EdU flow cytometry assay kit, following the manufacturer's protocol (Molecular Probes, Eugene, OR, USA). Cell cycle was analysed using a FACS Calibur flow cytometer (BD Biosciences, San Jose, CA, USA). Fifteen thousand events were evaluated using the Cell Quest (BD) software.

### Western Blotting

Total cell extracts were resolved on a SDS polyacrylamide gel, blotted on a Hybond P PVDF membrane (G&E Healthcare, UK). Membranes were blocked with PBST 5% non-fat dry milk, incubated with primary antibodies for 2h at room temperature, washed and hybridized for 1h at room temperature using the appropriate horseradish peroxidase-conjugated secondary antibody (rabbit and mouse, BioRad, Hercules, California, USA). Detection was performed with the ECL chemiluminescence kit (Perkin Elmer, Waltham, Massachusetts, USA). The following antibodies were used: anti-p63 (Ab4, Neomarkers, Fremont, California, USA; dilution 1:500), anti-K10 (Covance, Princeton, Nj, USA; dilution 1:1000), anti-β actin (Sigma, St Louis, Minnesota, USA; dilution 1:5000).

### Cell fractionation and RNA extraction

Cells were harvested by trypsinization and collected in 15-ml conical tubes on ice, washed three times with cold PBS, transferred to microfuge tubes, and pelleted at 4000 rpm for 3min in a refrigerated centrifuge (Eppendorf 5415R). Cell pellets were resuspended in 1ml of RSB (10mM Tris, pH 7.4, 10mM NaCl, 3mM MgCl2), incubated for 3min on ice, followed by centrifugation at 4°C. The volume of the swelled cell pellet was estimated and resuspended by slow pipetting with four times its volume of lysis buffer RSBG40 [10mM Tris, pH 7.4, 10mM NaCl, 3mM MgCl2, 10% glycerol, 0.5% Nonidet P-40, 0.5mM dithiothreitol (DTT), and 100U/ml rRNasin (Promega, WI)]. Nuclei were pelletted by centrifugation at 7000rpm for 3min, and the supernatant was recovered and saved as the cytoplasmic fraction. Nuclear pellets were resuspended in RSBG40, and one-tenth volume of detergent [3.3% (wt/wt) sodium deoxycholate and 6.6% (vol/vol) Tween 40] was added with slow vortexing, followed by incubation on ice for 5 min. Nuclei were again pelletted and the supernatant was pooled with the previous cytoplasmic fraction. Nuclear pellets were washed once more in RSBG40, collected at 10,000 rpm for 5min, and the resulting pellet used for nuclear RNA extraction. Cell lysis and nuclear integrity was monitored by light microscopy following trypan blue staining. RNA was extracted using the TRIZOL method, according to the manufacturer's instructions (Invitrogen, CA).

### Constructs and Luc Assay

Uc.291 genomic sequence (231 bp) was amplified by PCR from human genomic DNA using the following primers: uc.291pGLF 5'-ggccgctagcgggaacttatttgtatgcagc-3' (SEQ ID NO:2); uc.291pGLR 5'-ggccctcgagcaactgcagtgcctgcatgttttc-3' (SEQ ID NO:3). The 231-bp fragment, after NheI/XhoI restriction, was ligated to NheI/XhoI-linearized pGL3-promoter vector (Promega, Madison, WI, USA). A total of 1x10⁵ Saos-2 cells were seeded in 12-well dishes 24h before transfection. 600 ng of pGL3 vectors, 13 pmoles of uc.291 siRNA and 10 ng of *Renilla luciferase* pRL-CMV vector were cotransfected using Lipofectamine 2000 (Invitrogen). Luciferase activity of cellular extracts were measured 24h after transfection by using a Dual Luciferase Reporter Assay System (Promega); light emission was measured using an OPTOCOMP I luminometer. Efficiency of transfection was normalized using *Renilla* luciferase activity.

### In situ-hybridization

The sequence of the uc.291 probe is as follows:
/5DigN/ACAACCACATGGGCTATCAAGA/3Dig (SEQ ID NO:4); the U6 probe is from Exiqon, the Cat # is 99002-15. The formalin-fixed paraffin embedded tissue sections were dewaxed in xylenes, and rehydrated through an ethanol dilution series. Tissue sections were digested with 15 µg/mL proteinase K for 10 minutes at RT, were then loaded onto Ventana Discovery Ultra for *in situ* hybridization analysis. The tissue slides were incubated with Dig labeled mercury probe (Exiqon) for 2 hrs at 50 C. The digoxigenins can then be detected with a polyclonal anti-DIG antibody and Alkaline Phosphatase conjugated second antibody (Ventana) using NBT-BCIP as the substrate. The double-dig labelled control U6 snRNA probe is also from Exiqon.

### Results

### Uc.291 is expressed in keratinocytes during calcium-induced differentiation

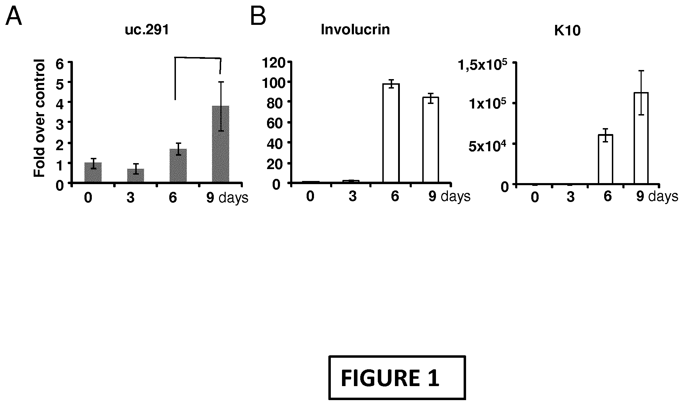

Primary human keratinocytes were induced to differentiate by calcium addition, for 3, 6 or 9 days. uc.291 expression was evaluated by real time RT-PCR. uc-291 expression is time dependent; the inventors used as positive controls the two differentiation markers involucrin and K10 (Figures 1A and 1B). *In situ*-hybridization (data not shown) confirmed that uc.291 is specifically expressed in the epidermis.

### Depletion of uc.291 delays differentiation

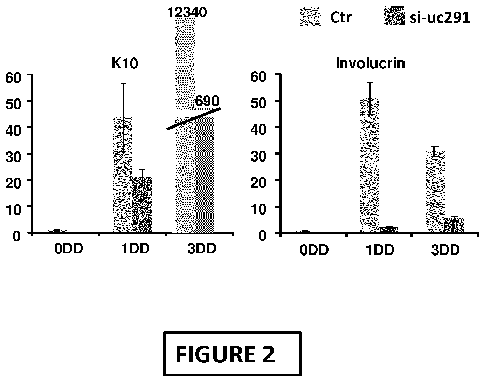

To confirm that uc.291 is important during keratinocyte differentiation, the inventors silenced uc.291 by siRNA in primary human keratinocytes and differentiation was induced by calcium addition. Evaluation of cell morphology in scramble transfected cells (Ctrl) and si-uc.291 transfected cells upon 1, 2, 3 days calcium addition indicate that si-uc.291 cells maintain longer the round shape, typical of proliferating cells, while Ctrl (scramble transfected cells) appear flat and elongated already after 1 day Calcium treatment (Figure 2). To better quantify this, the inventors have evaluated the expression of differentiation markers (K10 and involucrin) during differentiation in scramble transfected cells (Ctrl) and si-uc.291 transfected cells. The data obtained indicate that differentiation is strongly delayed by the absence of uc.291.

### Depletion of uc.291 increases proliferation

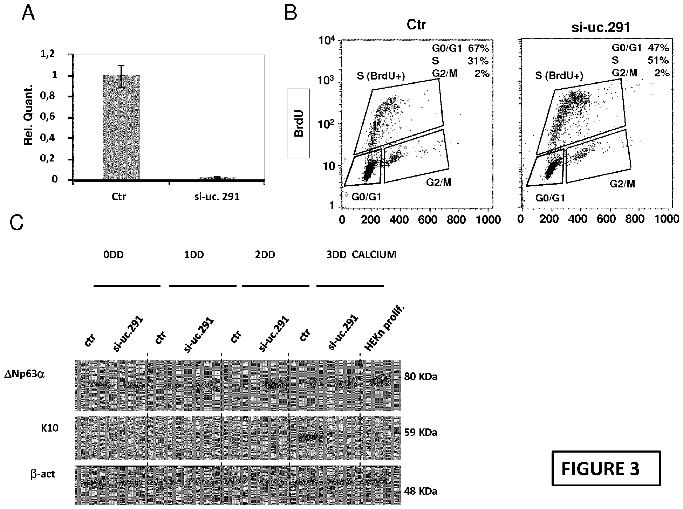

To evaluate the effect of uc.291 during proliferation, the inventors silenced uc.291 by siRNA in primary human keratinocytes. Silencing affects both long terminal proliferation, evaluated by clonogenic assay (Figure 3) and short term differentiation, evaluated by FACS analysis after 3 days calcium (1.2 mM) treatment. These results were also confirmed at protein level, evaluating by Western blot the proliferation marker (p63) and differentiation marker (K10) during calcium induced differentiation (1, 2, 3 days) upon uc.291 depletion (Figure 3C).

### Uc.291 is expressed in the nucleus and functions as transcriptional enhancer

In order to explore the molecular mechanism through which uc.291 affect proliferation and differentiation in keratinocytes, the inventors performed biochemical fractionation of keratinocyte cellular extract in order to separate nucleus from cytosol. Uc.291 is mainly detected in the nucleus, as shown in Figure 4A. Furthermore, to understand if it works as transcriptional enhancer, the inventors cloned the conserved sequence of uc.291 in a vector for luciferase-assay, upstream of the promoter, and transfected this vector (uc.291) and the control vector (Ctr) in Saos-2 cells that express endogenous uc.291. The inventors can observe that luciferase emission increases two folds over control (Figure 4B), indicating that uc.291 acts as transcriptional enhancer.

### SFTPD, ZMIZ1, ZSWIM8 genes are located in proximity of uc.291 locus and their expression parallels uc.291 expression

In order to identify a possible mechanism responsible for uc.291 effects in controlling proliferation and differentiation of keratinocytes, the inventors investigate the role of the gene located in proximity of uc.291 whose expression parallels uc.291 expression during differentiation. Among these genes the inventors found that SFTPD, ZMIZ1a and ZSWIM8 genes are highly expressed during keratinocytes differentiation (Figure 5A and 5B) and their mRNA increases similarly to uc.291 after calcium addition.

### Uc.291 expression enhances SFTPD, ZMIZ1, ZSWIM8 expression and silencing of SFTPD, ZMIZ1, ZSWIM8 recapitulate si-uc.291 effects in keratinocytes

To demonstrate that uc.291 expression acts as transcriptional enhancer for SFTPD, ZMIZ1 and ZSWIM8 the inventors silenced uc.291 and added calcium for three days. The inventors found a 50% reduction of SFTPD, ZMIZ1 and ZSWIM8 expression upon si-uc.291 (Figure 6A and 6B). Interestingly, si-SFTPD, si-ZMIZ1 and si-ZSWIM8 phenocopy uc.291 silencing in terms of cell morphology (not shown) and Involucrin expression. Indeed, si-uc.291 and si-SFTPD cells treated for three days with calcium have a similar morphology (round shape, not shown) as compared to three days calcium, in which the cells appear undergoing to differentiation. Also involucrin expression is delayed upon si-SFTPD, si-ZMIZ1 and si-ZSWIM8 (Figure 7).

### Different compounds can modulate uc.291 expression

The inventors have evaluated the effects of compounds (N1, 0.05%; N2, 1%) in regulating uc.291 expression level (Figures 9 and 10). The inventors observed that the compounds tested have synergic effects with calcium in enhancing uc.291 expression.

### Summary

The data presented indicate that:
- uc.291 is induced during calcium-driven keratinocyte differentiation. It is a pro-differentiation lnc-RNA.
- uc.291 is expressed in the upper layers of human epidermis.
- uc.291 knock-down, by siRNA, decreases keratinocytes differentiation (with differentiation markers: K10 and involucrin).
- uc.291 knock-down increases proliferation (short and long-term proliferation).
- uc.291 is mainly expressed in the nucleus of the keratinocytes.
- uc.291 is a transcriptional regulator with cis-enhancer activity for SFTPD, ZMIZ1 and ZSWIM8 genes. While the roles of ZMIZ1 and ZSWIM8 are not known in the epidermis so far, Surfactant protein D, encoded by SFTPD gene, is expressed in the epidermis where it shows potent immuno-modulatory and anti-inflammatory properties.

### Conclusion

These data suggest that uc.291 has dual functions in skin barrier function formation: it allows proper differentiation and keratinization and maintains skin homeostasis by preventing pathogen infection and having anti-inflammatory properties.

### Example 2: cosmetic compositions

The following compositions can be prepared in a manner conventional to those skilled in the art. The amounts indicated below are expressed as percentages by weight.

### O/W emulsion

| **INCI /TRADE NAME SUPPLIER** | **(% W/W)** |
|---|---|
| Jojoba esters | 1-10 |
| Meadowfoam seed oil | 1-10 |
| Butyrospermum Parkii Butter (LIPEX SHEA) | 1-10 |
| Butyrospermum parkii butter (LIPEX SHEASOFT) | 1-10 |
| Shea Butter Ethyl Esters (LIPEX SHEALIGHT) | 1-10 |
| Butyrospernum parkii butter extract (LIPEX SHEA TRIS) | 1-10 |
| Pentaerythrityl stearate/caprate/ caprylate/ adipate (SUPERMOL S-SO) | 0.5-5 |
| Diisostearyl dimer dilinoleate (SCHERCEMOL DISD) | 1-10 |
| Octyldodecyl myristate | 1-5 |
| Phytosqualan | 0.1-7 |
| Phytosteryl/octyldodecyl lauroyl glutamate (ELDEW PS-203) | 0.01-5 |
| Xanthan gum | 1-10 |
| Cetearyl alcohol & coco-glucoside | 1-5 |
| Hydroxyethyl acrylate/sodium acryloyldimethyltaurate copolymer & squalane & polysorbate 60 | 1-5 |
| Dimethicone & dimethicone crosspolymer | 1-5 |
| Dimethicone & dimethicone/vinyl dimethicone crosspolymer (KSG-016F) | 0.1-10 |
| Polymethylmethacrylate | 0.1-10 |
| Sodium hyaluronate | 0.01-3 |
| Glycerin | 1-30 |
| Polyquaternium-51 | 1-10 |
| Adenosine | 0.1-0.5 |
| Niacinamide | 0.1-5 |
| Tremella fuciformis polysaccharide | 0.1-5 |
| Palmitoyl Tripeptide-1 & Palmitoyl Tetrapeptide-7 | 1-5 |
| Secale Cereale (Rye) Seed Extract | 1-5 |
| Camellia leaf extract as obtained in example 1 | 0.001-5 |
| Stem cells of Camellia as obtained in example 1 | 0.001-5 |
| Vitamin C and derivatives | 0.001-5 |
| Glycols (Caprylyl Glycol and/or Pentylene Glycol and/or Butylene Glycol and/or propanediol) | 0,1-10 |
| Water | Qs 100 |

### Transparent aqueous serum

| **INCI /TRADE NAME SUPPLIER** | **(% W/W)** |
|---|---|
| Camellia kissi seed oil | 0.01-5 |
| PEG-40 hydrogenated castor oil | 0.01-5 |
| PPG-6 decyltetradeceth-30 | 0.01-5 |
| CARBOMER | 0.01-5 |
| Xanthan gum | 1-10 |
| Agar & xantham gum | 0.1-10 |
| Hydrogenated starch hydrolysate & maltooligosyl glucoside (MG-60) | 0.1-10 |
| Glycerin | 1-30 |
| Polyquaternium-51 | 1-10 |
| Adenosine | 0.1-0.5 |
| Niacinamide | 0.1-5 |
| Tremella fuciformis polysaccharide | 0.1-5 |
| Palmitoyl Tripeptide-1 & Palmitoyl Tetrapeptide-7 | 1-5 |
| Secale Cereale (Rye) Seed Extract | 1-5 |
| Camellia leaf extract as obtained in example 1 | 0.001-5 |
| Stem cells of camellia as obtained in example 1 | 0.001-5 |
| Vitamin C and derivatives | 0.001-5 |
| Glycols (Caprylyl Glycol and/or Pentylene Glycol and/or Butylene Glycol and/or propanediol) | 0,1-10 |
| Water | Qs 100 |

### O/W emulsion cream

| **INCI/TRADE NAME SUPPLIER** | **(% W/W)** |
|---|---|
| Jojoba esters | 1-10 |
| C13-16 ISOPARAFFIN | 1-10 |
| Acacia decurrens/jojoba/sunflower seed wax/polyglyceryl-3 esters (ACTICIRE) | 1-10 |
| Butyrospermum parkii butter (LIPEX SHEASOFT) | 1-10 |
| Shea Butter Ethyl Esters (LIPEX SHEALIGHT) | 1-10 |
| Butyrospernum parkii butter extract (LIPEX SHEA TRIS) | 1-10 |
| Pentaerythrityl stearate/caprate/ caprylate/ adipate (SUPERMOL S-SO) | 0.5-5 |
| Diisostearyl dimer dilinoleate (SCHERCEMOL DISD) | 1-10 |
| Octyldodecyl myristate | 1-5 |
| Cetyl alcohol & glyceryl stearate & peg-75 stearate & ceteth-20 & steareth-20 | 0.1-7 |
| CARBOMER | 0.01-5 |
| Xanthan gum | 1-10 |
| Dimethicone & dimethicone/vinyl dimethicone crosspolymer (KSG-016F) | 0.1-10 |
| Silica (SATINIER M5) | 0.1-10 |
| Sodium hyaluronate | 0.01-3 |
| Glycerin | 1-30 |
| Polyquaternium-51 | 1-10 |
| Adenosine | 0.1-0.5 |
| Niacinamide | 0.1-5 |
| Tremella fuciformis polysaccharide | 0.1-5 |
| Palmitoyl Tripeptide-1 & Palmitoyl Tetrapeptide-7 | 1-5 |
| Secale Cereale (Rye) Seed Extract | 1-5 |
| Camellia leaf extract as obtained in example 1 | 0.001-5 |
| Stem cells of camellia as obtained in example 1 | 0.001-5 |
| Vitamin C and derivatives | 0.001-5 |
| Glycols (Caprylyl Glycol and/or Pentylene Glycol and/or Butylene Glycol and/or propanediol) | 0,1-10 |
| Water | Qs 100 |

## Claims

1. *In vitro* method for screening for candidate compounds for improving skin barrier function, and/or for preventing and/or attenuating ageing of the skin, and/or for hydrating the skin, comprising the following steps:
a. bringing at least one test compound in contact with a sample of keratinocytes ;
b. measuring the expression or the activity of uc.291 in said keratinocytes;
c. selecting the compounds for which an activation of at least 20%, preferably at least 30%, preferably at least 40% of the expression or an activation of at least 20%, preferably at least 30%, preferably at least 40% of the activity of uc.291 is measured in the keratinocytes treated in a. compared with the untreated keratinocytes.

2. Method according to Claim 1, **characterized in that** step b. is performed before and after step a.

3. Method according to Claim 1, **characterized in that** it comprises the following steps:
a'. preparing at least two samples of keratinocytes ;
a. bringing one of the samples into contact with at least one test compound ; then
b. measuring the expression or the activity of uc.291 in said samples; and
c. selecting the compounds for which an activation of at least 20%, preferably at least 30%, preferably at least 40% of the expression or an activation of at least 20%, preferably at least 30%, preferably at least 40% of the activity of uc.291 is measured in the keratinocytes treated in a. compared with the sample of untreated keratinocytes.

4. Method according to any one of Claims 1 to 3, **characterized in that** the test compounds are chosen from botanical extracts.

5. Method according to any one of Claims 1 to 4, **characterized in that** the activation of expression or activity of uc.291 measured in step c. is of at least 50%, preferably of at least 60%.

## Patentansprüche

1. In vitro-Verfahren zum Screening auf Kandidatenverbindungen zum Verbessern der Hautbarrierefunktion und/oder zum Verhindern und/oder zum Abschwächen der Alterung der Haut und/oder zum Hydratisieren der Haut, umfassend die folgenden Schritte:
a. In-Kontakt-Bringen mindestens einer Testverbindung mit einer Probe von Keratinozyten;
b. Messen der Expression oder der Aktivität von uc.291 in den Keratinozyten;
c. Auswählen der Verbindungen, für welche eine Aktivierung von mindestens 20 %, vorzugsweise mindestens 30 %, vorzugsweise mindestens 40 % der Expression oder eine Aktivierung von mindestens 20 %, vorzugsweise mindestens 30 %, vorzugsweise mindestens 40 % der Aktivität von uc.291 gemessen wird in den in a. behandelten Keratinozyten im Vergleich mit den unbehandelten Kerationozyten.

2. Verfahren nach Anspruch 1, **dadurch gekennzeichnet, dass** Schritt b vor und nach Schritt a durchgeführt wird.

3. Verfahren nach Anspruch 1, **dadurch gekennzeichnet, dass** es die folgenden Schritte umfasst:
a'. Herstellen von mindestens zwei Proben von Keratinozyten;
a. In-Kontakt-Bringen einer der Proben mit mindestens einer Testverbindung; dann
b. Messen der Expression oder der Aktivität von uc.291 in den Proben; und
c. Auswählen der Verbindungen, für welche eine Aktivierung von mindestens 20 %, vorzugsweise mindestens 30 %, vorzugsweise mindestens 40 %der Expression oder eine Aktivierung von mindestens 20 %, vorzugsweise mindestens 30 %, vorzugsweise mindestens 40 % der Aktivität von uc.291 gemessen wird in den in a. behandelten Keratinozyten im Vergleich mit den unbehandelten Kerationozyten.

4. Verfahren nach einem der Ansprüche 1 bis 3, **dadurch gekennzeichnet, dass** die Testverbindungen aus Pflanzenextrakten ausgewählt werden.

5. Verfahren nach einem der Ansprüche 1 bis 4,**dadurch gekennzeichnet, dass** die Aktivierung von Expression oder Aktivität von uc.291, gemessen in Schritt c., mindestens 50 %, vorzugsweise mindestens 60 % ist.

## Revendications

1. Procédé *in vitro* pour le criblage de composés candidats pour l'amélioration de la fonction barrière de la peau, et/ou pour la prévention et/ou l'atténuation du vieillissement de la peau, et/ou pour l'hydratation de la peau, comprenant les étapes suivantes :
a. la mise en contact d'au moins un composé à tester avec un échantillon de kératinocytes ;
b. la mesure de l'expression ou de l'activité de l'uc.291 dans lesdits kératinocytes ;
c. la sélection des composés pour lesquels une activation d'au moins 20 %, de préférence d'au moins 30 %, de préférence d'au moins 40 % de l'expression ou une activation d'au moins 20 %, de préférence d'au moins 30 %, de préférence d'au moins 40 % de l'activité de l'uc.291 est mesurée dans les kératinocytes traités en a. comparativement aux kératinocytes non traités.

2. Procédé selon la revendication 1, **caractérisé en ce que** l'étape b. est effectuée avant et après l'étape a.

3. Procédé selon la revendication 1, **caractérisé en ce qu'**il comprend les étapes suivantes :
a'. la préparation d'au moins deux échantillons de kératinocytes ;
a. la mise en contact de l'un des échantillons avec au moins un composé à tester ; puis
b. la mesure de l'expression ou de l'activité de l'uc.291 dans lesdits échantillons ; et
c. la sélection des composés pour lesquels une activation d'au moins 20 %, de préférence d'au moins 30 %, de préférence d'au moins 40 % de l'expression ou une activation d'au moins 20 %, de préférence d'au moins 30 %, de préférence d'au moins 40 % de l'activité de l'uc.291 est mesurée dans les kératinocytes traités en a. comparativement aux kératinocytes non traités.

4. Procédé selon l'une quelconque des revendications 1 à 3, **caractérisé en ce que** les composés à tester sont choisis parmi des extraits botaniques.

5. Procédé selon l'une quelconque des revendications 1 à 4, **caractérisé en ce que** l'activation de l'expression ou de l'activité de l'uc.291 mesurée dans l'étape c. est d'au moins 50 %, de préférence d'au moins 60 %.


### SEQUENCE LISTING

<110> CHANEL PARFUMS BEAUTE
<120> ACTIVATORS OF uc.291 FOR USE FOR IMPROVING SKIN BARRIER FUNCTION AND/OR FOR PREVENTING AND/OR ATTENUATING SKIN AGEING AND/OR FOR HYDRATING SKIN
<130> BEP150417EP
<160> 4
<170> PatentIn version 3.5
<210> 1
   <211> 424
   <212> DNA
   <213> Homo sapiens
<400> 1
   cttatttgta tgcagcacaa atttcagaat ctgttctcag cctgtgccga gtgaaaaatg       60
   gcctgcattt tcttgatagc ccatgtggtt gtaaagaata aatggctaat gaattacaga      120
   tgaacattga cgcaaattaa tcttcccgct gtccctgggt tatatggcag ccatttaaaa      180
   gtttaatcaa tacactaaag ttgaaaacat gcaggcactg cagttgtttg gatgtaataa      240
   acatcagagg gaaccgggag gtttgcaccc agtccatgta tcataatgac aggtatttat      300
   gtttaatgga ctaaatattt tttattggaa gggagcaaat gtcagcttaa ctttgtgagc      360
   cccgcattca actttccttt gaggttggtg aaagacggct gacgtgtcat tggaaatgaa      420
   atgt                                                                   424
<210> 2
   <211> 31
   <212> DNA
   <213> Artificial Sequence
<220>
   <223> primer uc.291pGLF
<400> 2
   ggccgctagc gggaacttat ttgtatgcag c 31
<210> 3
   <211> 34
   <212> DNA
   <213> Artificial Sequence
<220>
   <223> primer uc.291pGLR
<400> 3
   ggccctcgag caactgcagt gcctgcatgt tttc 34
<210> 4
   <211> 22
   <212> DNA
   <213> Artificial Sequence
<220>
   <223> uc.291 probe
<400> 4
   acaaccacat gggctatcaa ga 22